(19) Europäisches Patentamt
European Patent Office
Office européen des brevets

(11) **EP 4 356 860 A1**

(12) **EUROPEAN PATENT APPLICATION**

(43) Date of publication:
24.04.2024 Bulletin 2024/17

(21) Application number: 22202949.8

(22) Date of filing: 21.10.2022

(51) International Patent Classification (IPC):
**A61B 34/20** (2016.01)

(52) Cooperative Patent Classification (CPC):
**A61B 34/20;** A61B 2017/00026; A61B 2017/00053;
A61B 2034/105; A61B 2034/2051; A61B 2576/023

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **TORBJøRN, Vik**
**Eindhoven (NL)**
• **SCHÄFER, Dirk**
**Eindhoven (NL)**
• **WILDEBOER, Rogier Rudolf**
**5656AG Eindhoven (NL)**

(74) Representative: **Philips Intellectual Property &
Standards**
**High Tech Campus 52**
**5656 AG Eindhoven (NL)**

(54) **MAPPING FUNCTION**

(57) A mechanism for adjusting a mapping function used to map a measurement by a fieldsensitive sensor to a position in a spatial co-ordinate system. A plurality of points in the spatial co-ordinate system (representing locations of one or more field sensitive sensors) are used to simulate image data. The simulated image data is compared to other image data of the same region to produce a similarity measure. The similarity measure is used to adjust the mapping function.

FIG. 3

**Description**

FIELD OF THE INVENTION

The present invention relates to the field of medical imaging, and in particular to the modelling of an anatomical cavity.

BACKGOUND OF THE INVENTION

Intrabody interventional devices are often used to investigate and treat anatomical cavities and anatomical elements within a subject or patient. For performing effective treatment, it is important for a clinician to be able to track the location of an interventional device.

One approach for tracking an interventional device is to provide the interventional device with one or more sensors, which can be labelled field-sensitive sensors. The measurements sampled by the field-sensitive sensor(s) caused by crossing electromagnetic fields induced in the subject change as the position of the interventional device changes. Examples of suitable measurements include a voltage measurement or a magnetic response (i.e., a measurement responsive to a change in a magnetic field). A single measurement may comprise a set of sub-measurements, such as a voltage measurement for each of a plurality of electromagnetic fields. The measurement changes can be used to track the location of the interventional device. One system describing such type of tracking for a catheter having multiple sensors is disclosed in the EP 3568068 A1.

It is possible to configure a processing system to determine a mapping function that maps from a measurement by a field-sensitive sensor to a point/position (e.g. definable using a set of spatial coordinates) in a spatial co-ordinate system that represents a true spatial relationship between the points. This mapping function can be alternatively labelled a measurement-to-position ("M2R") function. If the measurement is a voltage or voltage response, this function can be labelled a voltage-to-position ("V2R") function.

Typically, determination of the mapping function is performed by minimizing a cost function or loss function that parametrizes the mapping function and penalizes deviations between known inter-sensor distances and the estimated inter-sensor distances in the spatial co-ordinate system. This mapping can thereby take account of any field distortions within the body that results from different conductivities in different tissue types.

There is an ongoing desire to improve the accuracy and reliability of the mapping function.

SUMMARY OF THE INVENTION

The invention is defined by the claims.

According to examples in accordance with an aspect of the disclosure, there is provided a computer-implemented method of adjusting a mapping function configured to map from a measurement, of crossing electromagnetic fields, by a field-sensitive sensor within an anatomical cavity of a subject to a point in a spatial co-ordinate system.

The computer-implemented method comprises: receiving image data of a portion of the interventional device and/or a portion of the anatomical cavity; receiving a plurality of points in the spatial co-ordinate system, each point representing a location of a field-sensitive sensor in the spatial co-ordinate system, wherein each point is produced using the mapping function; generating simulated image data of the portion of the interventional device and/or the portion of the anatomical cavity by processing the plurality of points in the spatial co-ordinate system; determining a measure of similarity between the image data and the simulated image data; and adjusting the mapping function responsive to the determined measure of similarity.

The present disclosure proposes to provide a technique for modifying a mapping function that makes use of image data. The mapping function maps a measurement by a field-sensitive sensor to a position or point within a spatial co-ordinate system. The spatial co-ordinate system aim to represents a true spatial relationship between elements modelled or located therein. The spatial co-ordinate system may be a Euclidean space.

The image data is compared to simulated image data derived from points in the spatial co-ordinate system to produce a measure of similarity or "similarity measure". The points in the spatial co-ordinate system themselves represent different positions of one or more sensors.

The measure of similarity acts as an indicator of the accuracy of the mapping function. In particular, it has been recognized that the less accurate the mapping function, the less similar the simulated image data and the image data. Thus, the measure of similarity can perform as a cost function output for improving the mapping function.

The proposed approach is particularly advantageous if other systems or cost functions for modifying or tuning the mapping function are not available or are not accurate, e.g., if there is insufficient data to produce such a cost function.

It will be appreciated that the proposed method is entirely passive, and does not require any direct interaction with the subject or individual. Rather, historic data can be processed.

The image data may contain a representation of a portion of the interventional device within the anatomical cavity, e.g., at a first point in time. The simulated image data may contain a simulated representation of the same portion of the interventional device at or near the first point in time. Thus, the simulated image data may be generated from a plurality of points produced (using the mapping function) from one or more measurements originally sampled at or near the first point in time. This technique provides a reliable mechanism for generating image data that represents one another.

Thus, the image data may be of the portion of the interventional device and the step of generating simulated image may comprise generating simulated image data of the portion of the interventional device, e.g., which carries a plurality of field-sensitive sensors within the anatomical cavity.

The step of determining a measure of similarity may comprise processing the image data and the simulated image data using a cross-correlation technique. The cross-correlation technique may, for instance, be a normalized cross-correlation.

In some examples, the method further comprises receiving a cost function output, the cost function output being responsive to, for each of one or more pairs of points in the spatial co-ordinate system, a difference between the distance in the spatial co-ordinate system between the pair of points and a known distance between the pair of points. The step of adjusting the mapping function may be further responsive to the cost function output.

Thus, it is possible to combine the traditional system for modifying a mapping function (using the cost function output) with the proposed approach that makes use of a similarity measure. Use of both measures for modifying the mapping function can result in improved speed and efficiency of arriving at an accurate mapping function.

In some embodiments, each pair of points is a pair of points derived using the mapping function to process measurements, of the crossing electromagnetic fields, sampled by a pair of field-sensitive sensors at a same time; and a distance between the pair of field-sensitive sensors is predetermined (i.e., known in advance).

The step of adjusting the mapping function may comprise adjusting the mapping function responsive to a combination of the measure of similarity and the cost function output.

The step of adjusting the mapping function may comprises adjusting the mapping function to minimize or reduce the value of the combination of the measure of similarity and the cost function output. This a minimization approach can be used to modify the mapping function.

The combination of the measure of similarity and the cost function output may be a weighted combination. This allows to control how much each of the input values contributed to the combination, allowing (for instance) for less reliable or relevant value to be less influential on the value of the combination than other values.

The weighting provided to the measure of similarity may be configured to increase responsive to the number of the pairs of points in the spatial co-ordinate system being below a predetermined threshold number. Thus, when there are only few points available, the mapping function may be more influenced by the comparison between image data and simulated image data, as this may prove to be more accurate and provide better modification to the mapping function.

In some examples, the method further comprises receiving a time difference, being a difference between a time at which the plurality of points was originally sampled and a time at which the image data was originally obtained, wherein: the weighting provided to the measure of similarity is configured to reduce responsive to the time difference increasing.

The method may comprise receiving a plurality of measurements, of the electromagnetic electrical fields, by one or more field-sensitive sensors within the anatomical cavity; processing each measurement using the mapping function to produce a plurality of points in the spatial co-ordinate system; and generating an anatomical model of at least the portion of the anatomical cavity by processing the plurality of points in the spatial co-ordinate system.

The method may further comprise spatially registering the anatomical model and the image data responsive to the measure of similarity.

There is also proposed a computer-implemented method of mapping a measurement, of crossing electromagnetic fields, by a field-sensitive sensor within an anatomical cavity of a subject to a point in a spatial co-ordinate system.

The computer-implemented method comprises: obtaining the mapping function adjusted by performing any previously described method; and mapping the measurement by the field-sensitive sensor to the point in the spatial co-ordinate system using the obtained mapping function.

There is also proposed computer program product comprising code which, when executed by a processor circuit, causes the processor circuit to perform the steps of any herein described method. There is also proposed a non-transitory computer-readable medium or data carrier comprising or carrying the computer program product.

There is also proposed a processing system for adjusting a mapping function configured to map from a measurement, of crossing electromagnetic fields, by a field-sensitive sensor carried by an interventional device within an anatomical cavity of a subject to a point in a spatial co-ordinate system.

The processing system comprises an input interface and a data processor communicatively coupled to the input interface.

The input interface is configured to: receive image data of a portion of the interventional device and/or a portion of the anatomical cavity; and receive a plurality of points in the spatial co-ordinate system, each point representing a location of a field-sensitive sensor in the spatial co-ordinate system, wherein each point is produced using the mapping function.

The data processor is configured to: generate simulated image data of the portion of interventional device and/or the portion of the anatomical cavity by processing the plurality of points in the spatial co-ordinate system; determine a measure of similarity between the image data and the simulated image data; and adjust the mapping function responsive to the determined measure of similarity.

There is also proposed a mapping system for mapping a measurement, of crossing electromagnetic fields, by a field-sensitive sensor within an anatomical cavity of a subject to a point in a spatial co-ordinate system. The mapping system may be configured to obtain the mapping function adjusted by the processing system and map the measurement by the field-sensitive sensor to the point in the spatial co-ordinate system using the obtained mapping function.

The mapping function may itself comprise the processing system. Thus, the processing system may form an aspect of the overall mapping system.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying schematic drawings, in which:

Fig. 1 illustrates a system in which embodiments can be employed;
Fig. 2 illustrates an approach for generating an anatomical model;
Fig. 3 illustrates a method according to an embodiment;
Fig. 4 illustrates another method according to an embodiment;
Fig. 5 illustrates a processing system; and
Fig. 6 illustrates additional details of the processing system.

DETAILED DESCRIPTION OF THE EMBODIMENTS

The invention will be described with reference to the figures.

The detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The figures are merely schematic and are not drawn to scale. The same reference numerals are used throughout the figures to indicate the same or similar parts.

The invention provides a mechanism for adjusting a mapping function used to map a measurement by a field-sensitive sensor to a position in a spatial co-ordinate system. A plurality of points in the spatial co-ordinate system (representing locations of one or more field sensitive sensors) are used to simulate image data. The simulated image data is compared to other image data of the same region to produce a similarity measure. The similarity measure is used to adjust the mapping function.

Embodiments are based on the realization that a similarity between simulated image data (produced using the mapping function) and image data of a same region represents an accuracy of the mapping function. Thus, a similarity measure can be used as a relatively reliable error value in adjusting the mapping function.

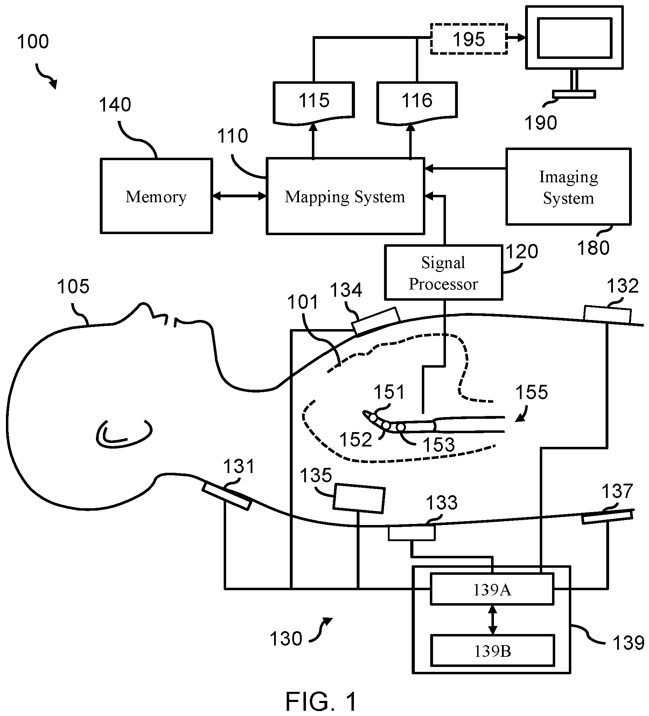

Fig. 1 illustrates a system 100 for analyzing or investigating an anatomical cavity 101 of a subject 105. The system 100 is configured to make use of approaches proposed by the present disclosure.

The anatomical cavity 101 may, for instance, be an anatomical cavity within any suitable anatomical element of the subject 105, such as the heart, liver, kidney, lungs etc. or parts thereof. Preferably, the cavity 101 is an atrium or ventricle of the heart.

An interventional device 150 of the system 100 carries one or more field-sensitive sensors 151, 152, 153. Each field-sensitive sensor 151, 152, 153 can be used to define or determine measurements that each represent a position of the interventional device 150 within the anatomical cavity 101. The interventional device 150 may be or comprise a catheter, such as an electrophysiological mapping catheter or an ablation catheter, although other devices or catheters may be used.

More particularly, a field-sensitive sensor produces a measurement that differs depending upon the position of said field-sensitive sensor within one or more crossing electromagnetic fields induced within the subject, and therefore depending upon the position of said field-sensitive sensor within the anatomical cavity 101. The measurement may comprise one or more data points, each data point being a response of the field-sensitive sensor to a particular electromagnetic field.

Any raw signals at field-sensitive sensor(s) 151, 152, 153 may be filtered, sampled and/or digitized using a signal processor 120 of the system 100 to produce the measurement. In particular, the signal processor 120 may (electrically) connect to the field-sensitive sensor(s) 151, 152, 153 and process the raw signals at the field-sensitive sensor(s) to produce a measurement for digital processing by a processing system or other similar device. The signal processor 120 may therefore comprise appropriate circuitry for performing such functions, e.g., filtering circuitry, sampling circuitry and/or digitizing circuitry (e.g., an analogue-to-digital converter).

One example of a suitable measurement is a set of voltages induced at the field-sensitive sensor by external electromagnetic fields induced in the subject. Thus, in some examples, the field-sensitive sensor is an electrode ("device electrode") at which a set of voltages, containing a voltage for each electromagnetic field, differs depending upon the position of said field-sensitive sensor with respect to the electromagnetic fields. In this scenario, the signal processor 120 may be able to sample and/or filter a set of voltages at each field-sensitive sensor to produce the measurement.

Another example of a suitable measurement is a magnetic response at the field-sensitive sensor to electromagnetic fields induced in the subject. Thus, in some examples, the field-sensitive sensor is a magnetic sensor (such as a Hall-effect sensor or a set of coils) at which a magnetic response of the magnetic sensor differs depending upon the position of said sensor with respect to the electromagnetic field. In this scenario, the signal processor 120 may be able to sample and/or filter the voltage(s) (or other electrical response) produced by each magnetic sensor to produce the measurement.

The signal processor 120 may be appropriately configured for producing the measurements, e.g., to sample and/or filter the raw sensor signals produced by the field-sensitive sensors for digital processing.

The system 100 may comprise an electromagnetic field generating arrangement 130 for generating the electromagnetic field(s). An electromagnetic field generating arrangement 130 comprises a set of field generators 131, 132, 133, 134, 135, 137 operably connected to a field controller 139.

The arrangement 130 may be configured to generate crossing electromagnetic fields within at least a part of subject 105 using the set of field generators positioned externally with respect to the subject 105. The field generators may include electrodes (for generating electric fields) or solenoids (for generating magnetic fields). Other suitable examples will be apparent to the skilled person.

The field controller 139 is configured to control characteristics (e.g., voltage and/or current) of the electric signals provided to each field generator 131, 132, 133, 134, 135, 137 to thereby generate the crossing fields. For example, the arrangement may generate alternating electromagnetic fields as the crossing electromagnetic fields.

The field controller 139 may comprise a suitable signal generator 139A for generating and transmitting signals to each field generator 131, 132, 133, 134, 135, 137. The operation of the signal generator 139A may be controlled by a field controller processing system 139B of the field controller 139 (e.g., which may form part of any other processing circuitry of the system 100).

To distinguish between different electromagnetic fields (in the measurement taken at a field-sensitive sensor), each electromagnetic field may be generated with a different and distinguishable frequency and/or temporal code. A data point produced by the field-sensitive sensor may be an average or instantaneous measurement or response at the field-sensitive sensor to a particular frequency and/or temporal code, corresponding to the frequency and/or temporal code of the corresponding electromagnetic field. The combination of the data points can provide the measurement. Thus, a single measurement may comprise a plurality of data points.

The field controller 139 may be configured to control the electromagnetic fields to operate in the frequency range of 10 kHz to 1 MHz, preferably in the range of 10-100 kHz. This frequency range is particularly useful for ensuring penetration of a subject, whilst providing a frequency range that attenuates in human tissue without significant damage/injury to the tissue.

A reference sensor (e.g., 137) can serve, if required for the type of measurement, as a reference, e.g., a measurement reference, for all measurements taken by sensors, e.g., as a reference for the response of the field-sensitive sensors.

It will be apparent that the measurement of the field-sensitive sensor will change as the relative position of the field-sensitive sensor(s) move(s) about the subject. Such movement may be caused by a physician deliberately roving or moving the field-sensitive sensor within the cavity 101, to collect measurements at different locations within the cavity 101.

The principle of generating measurements using one or more field-sensitive sensors thereby facilitates tracking of the relative position of the interventional device 150 using a mapping system 110 that monitors the measurements of any field-sensitive sensor. The measurements are obtained or contained within a measurement coordinate system, sometimes referred to as a "measurement space".

In approaches where each measurement comprises one or more data points, and each data point is a response of the field-sensitive sensor to a particular electromagnetic field, it may be necessary to map the measurement to a spatial position in a spatial co-ordinate system, sometimes referred to as "R-space". The spatial co-ordinate system is a Euclidean space. This need is due to the inherent inhomogeneity of the electromagnetic field(s) in the subject, meaning that the distance between different measurements (in the measurement space) may not accurately represent a true distance. A spatial co-ordinate system is one that represents a true spatial relationship (e.g., proportionally scaled to geometric/physical distances) between elements in the spatial co-ordinate system.

Mapping can be performed using a suitable mapping function, sometimes called a measurement-to-position or "M2R function". If the measurement is a set of one or more voltages, this function can be labelled a voltage-to-position ("V2R") function. The mapping function therefore converts a measurement in a measurement coordinate system into a location or position (e.g., set of co-ordinates) in a spatial co-ordinate system. This mapping function will therefore determine the relative recorded position(s) of the sensors, and therefore of the interventional device, in a spatial co-ordinate system (e.g., compared to a measurement co-ordinate system).

Conventional approaches for generating such a mapping function are well established in the art. Example processes are disclosed by the European Patent Applications EP 0775466 A2, EP 3568068 A1 and EP 3607879 A1. Typically, determination of the mapping function is performed by minimizing a cost function that parametrizes the mapping function and penalizes deviations between known inter-sensor distances and the estimated inter-sensor distances in the spatial co-ordinate system.

Historically, generating a mapping function makes use of known inter-sensor distances to calibrate for the inhomogeneity in the electromagnetic fields. Thus, it is typically necessary to know the distance between each of the field-sensitive sensors. Historically, these have been provided by the manufacturer or operator of the interventional device.

Generation and/or modification of the mapping function may be performed by the mapping system 110. Thus, the mapping system may be configured to generate a mapping function for mapping a measurement, by a field-sensitive sensor, to a location in a spatial co-ordinate system. The mapping system 110 is thereafter able to map a measurement by the field-sensitive sensor to a location in the spatial co-ordinate system using the mapping function.

Once a number of locations of the interventional device and/or one or more sensors on the interventional device in the spatial co-ordinate system have been established, it is possible to construct an anatomical model 115 of the anatomical cavity 101. This process may be performed by the system 100, or a subsystem thereof such as the mapping system 110.

A brief description of how to generate an anatomical model is provided for improved contextual understanding. This description also aids to emphasize a particular use-case scenario for data derived from a measurement-to-position function, and therefore the clinical benefit(s) and advantage(s) of a more accurate measurement-to-position function.

Broadly, the mapping system 110 may build an anatomical model 115 of a portion of the anatomical cavity 101 (e.g., a chamber, vessel or void) within a subject by processing a cloud of points. Each point represents a location or position of the interventional device or sensor in the spatial co-ordinate system, and could therefore be represented as a set of data, e.g., a set of co-ordinates.

More specifically, a reconstruction algorithm is used to generate an anatomical model of the (investigated part of the) anatomical cavity. The anatomical model may, for example, be a 3D (polygon) mesh or surface that depicts or models the (known bounds of the) anatomical cavity. In the present disclosure, the anatomical model preferably comprises a 3D mesh defined by a plurality of vertices, each vertex being associated with a different location in the predefined co-ordinate system. The 3D mesh is therefore defined in the same co-ordinate system as the determined locations of the field-sensitive sensor(s). The 3D mesh may, for instance, be defined as sets of co-ordinates defining vertices of the 3D mesh.

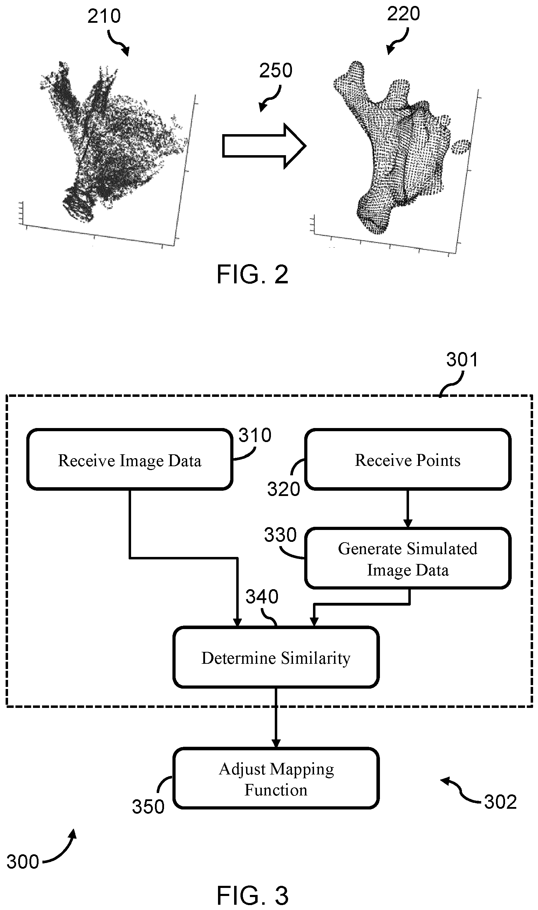

The process of reconstructing an anatomical model from a point cloud is conceptually illustrated in Fig. 2, which demonstrates a process 250 in which a cloud of points 210 ("point cloud") is transformed into an anatomical model 220. In the illustrated example, this is performed by creating a (3D) surface from the point cloud data, methods of which will be readily apparent to the skilled person.

For example, a point cloud can be converted into a polygon mesh or triangular mesh model (or other surface model) using a surface reconstruction approach. A variety of suitable approaches is discussed in Berger, Matthew, et al. "A survey of surface reconstruction from point clouds." Computer Graphics Forum. Vol. 26. No. 1. 2017, and further mechanisms will be readily apparent to the skilled person.

Thus, it is possible to generate an anatomical model 115 of the anatomical cavity 101 using measurements obtained using the field-sensitive sensor(s). More particularly, the mapping system 110 is able to map measurements captured using the field-sensitive sensor(s) to points in the spatial co-ordinate system (a Euclidean space) using the mapping function. The mapped points may then be processed, e.g., by the mapping system 110, to construct or generate the anatomical model.

Referring to Fig. 1, the anatomical model may be stored, for instance, in a memory 140 or storage unit. Of course, the anatomical model may be output for further processing and/or to a user interface 190, such as a screen or display, configured to provide a visual representation of the anatomical model.

Thus, the system 100 may comprise a user interface 190 configured to provide a visual representation of the anatomical model. In some examples, a display processor 195 may be configured to receive the anatomical model 115 from the mapping system 110 and render the anatomical model to generate display data that can be received and processed by the user interface 190 for providing a visual representation of the anatomical model.

More particularly, once a 3D mesh has been generated, then a visual representation of the 3D mesh can be provided. Providing a visual representation of the 3D mesh may comprise, for example, rendering a reconstruction of the surface of the (known) anatomical cavity. The visual representation may be provided to a user with an appropriate output device such as a display device. The system 100 may include the appropriate output terminals connectable to or connected to the output device according to known principles.

In one method, the steps of gathering measurements and constructing an anatomical model using the gathered measurements can be repeated until an acceptable anatomical model has been obtained.

Once an anatomical model has been generated, it is possible to monitor the location of the field-sensitive sensor(s) and/or interventional device with respect to the anatomical model. For example, both the model and the tracking of location of the device may have the same shared spatial coordinate system. The system 100 or the mapping system 110 may be accordingly configured to determine a location 116 of the field-sensitive sensor(s) and/or the interventional device. This may comprise identifying or determining one or more (possibly new) locations of each field-sensitive sensor and/or interventional device. This may include sampling measurements over time and mapping such additional measurements to respective additional locations in R-space using the mapping function. A model of the interventional device comprising the known locations of the field-sensitive sensors on the device may be used to generate a location 116 of at least a part of (e.g. preferably the part comprising one or more of the sensors) the interventional device.

The anatomical model in combination with monitored additional (new) locations may be used, for instance, to provide a visual representation of the known bounds of the anatomical cavity (represented by the anatomical model) and the relative position of the interventional device and/or its sensor(s) with respect to the anatomical cavity. This provides useful clinical information to an operator of the interventional device, e.g., to track the progress (e.g. location and/or orientation) of the interventional device and/or view the shape/structure/size of the anatomical cavity in which the interventional device is to be moved or is moving.

A generated anatomical model and/or mapping function may also/otherwise be used to generate one or more electrical maps of the anatomical cavity. In particular, the interventional device 155 may be configured to monitor one or more electrical measurements of the anatomical cavity or the bounds thereof (such as impedance, conduction, voltage, location activation values, or the like). The location at which each electrical measurement was obtained is derivable by mapping, for each electrical measurement, one or more measurements of the field-sensitive sensor(s) captured at a same time as the electrical measurement to a location in the shared spatial co-ordinate system. The corresponding electrical measurement(s) can then be plotted in the spatial co-ordinate system, e.g., projected onto the anatomical model, to generate an electrical map or model of the anatomical cavity.

This procedure may be performed by the mapping system or another processing system.

Example systems that make use of such approaches are known, including those produced by Biosense Webster and/or EnSite.

It will be appreciated that, in practice, many of the elements of the system 100 may be incorporated into a single device or computing system/arrangement. For instance, the signal processor 120, the mapping system 110 and/or the field controller 139 may be incorporated into a single computing system, such as a workstation.

Thus, there is provided a computing system comprising the mapping system 110, the signal processor 120 and/or the field controller.

The present disclosure provides an improved technique for modifying or adjusting the mapping function to improve its accuracy and speed of generating an accurate mapping function.

As previously explained, atypical mapping function is generated by adjusting the mapping function based on a difference between a predicted distance between two points (each representing the position of a different field-sensitive sensor for a same position of the interventional device) in the spatial co-ordinate system and a known distance.

The present disclosure proposes to process points in the spatial co-ordinate system, that were produced using the mapping function, to produce simulated image data. A measure of similarity between the simulated image data and (true) image data of a portion of the interventional device and/or a portion of the anatomical cavity is then produced. This measure of similarity is used to adjust the mapping function, e.g., provide or contribute to a loss/cost function output for adjusting the mapping function.

The measure of similarity may thereby act or contribute to a loss/cost function output for modifying the mapping function.

With reference to Fig. 1, the present disclosure can thereby provide a modification to the mapping system for facilitating improved generation of a mapping function.

Fig. 3 is a flow chart illustrating a method 300 according to an embodiment. The method 300 may be carried out by the mapping system 110 illustrated in Fig. 1. Continued reference will be made, where appropriate to the elements illustrated in Fig. 1.

The method 300 comprises a step 310 of receiving image data of a portion of the interventional device and/or a portion of the anatomical cavity. The image data may be generated by an imaging system 180, which may form part of the overall system 100 or may be a separate element.

The image data may, for instance, be X-ray, ultrasound or intracardiac echocardiography (ICE) image data of the portion of the anatomical cavity. However, any other suitable imaging data could be used to advantage (e.g., MRI, PET or the like). X-ray and ultrasound image data is particularly preferably, as this data can be obtained or sampled in a short time period. The image data is generated independently of any measurements taken by the field-sensitive sensor(s). Preferably, the image data is obtained non-invasively.

The method 300 further comprises a step 320 of receiving a plurality of points in the spatial co-ordinate system, each point representing a location of a field-sensitive sensor in the spatial co-ordinate system, wherein each point is produced using the mapping function (e.g., by the mapping system 110).

Thus, each point represents a location (in the spatial co-ordinate system) that has been derived, using the mapping function, from a measurement captured by a field-sensitive sensor, carried by an interventional device, within the anatomical cavity.

The method 300 also comprises a step 330 of generating simulated image data of a portion of the interventional device and/or the portion of the anatomical cavity by plurality of points. The simulated image data may, for instance, be a simulation of the anatomical cavity itself and/or a simulation of the interventional device within the anatomical cavity.

The image data obtained in step 310 corresponds to the simulated image data, i.e., contains a representation of the same target structure as the simulated image data. Thus, if the simulated image data comprises a representation of the interventional device within the anatomical cavity, then the image data (obtained in step 310) also contains a representation of the interventional device (that carries the field-sensitive sensor) within the anatomical cavity.

The method 300 also comprises a step 340 of determining a measure of similarity between the image data and the simulated image data.

The step 340 of determining a measure of similarity between the image data and the simulated image data can comprise using any suitable image similarity function. Suitable examples include those that use a cross-correlation algorithm, such as a normalized cross-correlation, to compare the image data and the simulated image data to produce the measure of similarity.

Other suitable examples of an image similarity function include those disclosed by: Unnikrishnan, Ranjith, and Martial Hebert. "Measures of similarity." 2005 Seventh IEEE Workshops on Applications of Computer Vision (WACV/MOTION'05)-Volume 1. Vol. 1. IEEE, 2005; Rohde, Gustavo Kunde, Carlos A. Berenstein, and Dennis M. Healy Jr. "Measuring image similarity in the presence of noise." Medical Imaging 2005: Image Processing. Vol. 5747. SPIE, 2005; or Van Heel, Marin. "Similarity measures between images." Ultramicroscopy 21.1 (1987): 95-100.

Of course, one or both of the image data and the simulated image data may undergo further image processing before being compared. This image processing may be used, for instance, to highlight or emphasize features considered to be common or present in one or both sets of image data.

In particular, the image processing may be configured to cause the image data and simulated image data to have similar statistical characteristics, for improved accuracy in comparing. One particularly suitable approach is disclosed by Haase, Christian, et al. "Model based 3D CS-catheter tracking from 2D X-ray projections: Binary versus attenuation models." Computerized medical imaging and graphics 38.3 (2014): 224-231.

In some examples, steps 310-340 can be adapted to comprise generating a plurality of measures of similarity. This can be achieved using multiple different image similarity functions to generate a plurality of measures of similarity between the same image data and simulated image data. As another example, this can be achieved by obtaining multiple instances of image data and corresponding instance simulated image data, and determine at least one measure of similarity between each instance of image data and its corresponding instance of simulated image data.

The method 300 also comprises a step 350 of adjusting the mapping function responsive to the determined measure of similarity. Step 350 may comprise modifying one or more parameters of the mapping function to minimize or reduce an error value, which can be at least partially defined using the determined measure of similarity.

Generally, a mapping function is defined by its parameters, e.g., weights, coefficients or "constants" that, if modified, change the value of an output for a same input. Modifying the parameters of the mapping function thereby modifies the mapping function to cause it to provide a different output. The parameterization of a mapping function can be represented by the symbol $\theta$ (which represents the various weights, parameters and/or coefficients used in the mapping function).

If steps 310-340 generates a plurality of measures of similarity, then step 350 may be correspondingly adapted to adjust the mapping function responsive to each determined measure of similarity. For instance, step 350 may comprise modifying one or more parameters of the mapping function to minimize or reduce an error value, which can be at least partially defined using each determined measure of similarity.

More generally, the method 300 can be divided into (at least) two processes. A similarity measure generation process 301 (comprising steps 310, 320, 330 and 340) and a mapping function adjustment process 302 (comprising step 350).

The method 300 can be iteratively repeated, e.g., to iteratively adjust and/or update the mapping function (e.g., with an aim of reducing or minimizing a loss value generated using at least the determined measure of similarity.

A first example approach for performing the similarity measure generation process 301 is hereafter described.

In this first example approach, the plurality of points received/obtained in step 320 comprises (only) one or more sets of points. Each point in a same set of points represents a point for a different field-sensitive sensor of the interventional device.

Each set of points comprises only a single point for each field-sensitive sensor of the interventional device(s). Each point represents the position of that field-sensitive sensor at a same point in time and/or without a movement of any of the field-sensitive sensors (i.e., for a same pose and shape of the interventional device(s)). Pose refers to a position and orientation of an element.

Thus, each set of points comprises only points derived (using the mapping function) from measurements captured by a plurality of field-sensitive sensors mounted on one or more interventional devices. For each set of points, the measurements are captured only when the interventional device(s) are at a respective fixed or particular position, i.e., and are not moved whilst the measurements are captured. Thus, each point represents the location of a different field-sensitive sensor mounted upon one or more interventional devices.

Put another way, the points in the set of points are derived from a measurements obtained/sampled without the interventional device(s) moving between sampling each measurement. In this way, the plurality of points effectively represent the pose of the interventional device(s) at a particular point in time.

Thus, each set of points is associated with a particular position or time point j. This represents the position of the interventional device(s) at a particular point in time. There may be J sets of points (i.e., J different positions of the interventional device).

In this first example approach, the image data received in step 310 comprises, for each set of points, image data containing a representation of the one or more interventional devices at their respective fixed positions (i.e., at the (same) particular point in time associated with the set of points).

Thus, if there are multiple sets of points obtained in step 320, there may be multiple instances of image data. Each set of points temporally corresponds to an instance of image data. In particular, each set of points corresponds to image data depicting the interventional device(s) having the same pose (i.e., position and orientation) as when the corresponding set of points was sampled.

Thus, if there are j sets of points, there are j instances of image data (each corresponding to the same position for the interventional device).

The step 330 of generating simulated image data may comprise simulating a projection of the one or more interventional devices. Thus, the simulated image data contains a representation of a portion of the interventional device(s). Step 330 may comprise simulating image data for each set of points. Thus, an instance of simulated image data is generated for each instance of obtained image data.

Approaches for simulating image data that simulates a projection of the one or more interventional devices within an anatomical cavity are known. One example approach is described in Haase, Christian, et al. "Model based 3D CS-catheter tracking from 2D X-ray projections: Binary versus attenuation models." Computerized medical imaging and graphics 38.3 (2014): 224-231. This same document also discloses approaches for performing image processing on the image data and/or simulated image data, as well as one possible approach for generating a similarity measure between image data and simulated image data, which can be used for step 340.

Step 340 can be carried out using any previously described approach (i.e., using any image similarity function).

In particular, an initial similarity measure may be generated for each set of points (and therefore each instance of corresponding simulated image data and image data) using any previously described image similarity function. If more than one initial similarity measure is generated, then these may be combined (e.g., summed or averaged) to produce the measure of similarity.

In some examples, each initial similarity measure may be weighted or biased, e.g., such that some initial similarity measures contribute to or influence the measure of similarity more than others.

The magnitude of the weighting (for any given initial similarity measure) may be responsive to a difference ("time difference") between the time at which the plurality of measurements (used to produce the set of points) were originally sampled and the time at which the corresponding image data was originally sampled. In particular, the larger this difference, the greater the discrepancy between the assumed device position (in the simulated image data) and the device position in the (actual) image data. This is because there will be some inherent motion in the individual (e.g., due to a heartbeat or the like) that affects the relationship between the simulated image data and the (true) image data, which is not attributable to the mapping function.

The value of the time difference can be determined if, for instance, the image data and the plurality of measurements (for the corresponding set of points) are each associated with a timestamp. A difference between the two timestamps may represent the time difference.

Thus, in some examples, the greater the time difference, the lower the weighting/contribution of the initial similarity measure to the measure of similarity.

Other reasons or motivations for changing a weighting or contribution of different initial similarity measures will be apparent to the skilled person.

For instance, initial similarity measures derived using measurements obtained in a more distant past may be weighted so as to contribute less to the measure of similarity than more recent initial similarity measures.

Mathematically, one embodiment for the first example approach for performing the similarity measure generation (to produce a measure of similarity $B(\theta)$) can be represented as:

$$B(\theta) = \sum_{j \in J} Y_j . G(g(I_j), P_j\left(M(f(\mathbf{x}^j; \theta))\right)) \tag{1}$$

where:

j is a time point at which the image data was sampled/produced;

J is an index set of all such time points;

G(.) is an image similarity function (such as one that employs a cross-correlation technique);

g(.) is an optional image processing technique (such as a logarithmic and/or top-hat filter);

$P_j(.)$ is an image simulation technique, such as a ray projection technique;

M(.) is an optional volumetric model of device attenuation coefficients;

f(.) is the mapping function;

$\mathbf{x}^j$ is a plurality of measurements obtained by a respective plurality of field-sensitive sensors mounted on one or more interventional device, each of which are at a particular position at time point j;

$Y_j$ is an optional weighting or biasing value; and

$\theta$ is the parameterization of the mapping function.

In this way, $f(\mathbf{x}^j; \theta)$ represents a set of points obtained in step 320. $I_j$ or $g(I_j)$ represents the image data obtained in step 310. $P_j(.)$ defines the simulated image data.

The value of J (i.e., the number of time points) may be any integer value, e.g., only 1 or more than 1. As an example, the value of J may be no less than 5, e.g., no less than 10. The greater the value of J, the more representative the measure of similarity is to the true accuracy of mapping function, e.g., to account for noise or minor/negligible errors in the mapping function.

$G(g(I_j), P_j(M(f(\mathbf{x}^j; \theta))))$ represents an initial similarity measure. These are combined to produce the similarity measure $B(\theta)$.

As previously explained, each initial similarity measure may be weighted or biased during the combination procedure (represented by the sigma symbol in equation (1)). In equation (1), this weighting or biasing is defined using a weighting value $Y_j$.

The value of the weighting value $Y_j$ may be responsive to a difference between the time at which the plurality of measurements $\mathbf{x}^j$ (used to produce the set of points $f(\mathbf{x}^j; \theta)$) were originally sampled and the time at which the corresponding image data $g(I_j)$ was originally sampled. In particular, the larger this difference, the greater the discrepancy between the assumed device position (in the simulated image data) and the device position in the (actual) image data. This is because there will be some inherent motion in the individual (e.g., due to a heartbeat or the like).

In other words, it is recognized that there may, in practice, be a discrepancy between the time at which the plurality of measurements are sampled and the time at which the image data is obtained (even if these are both represented by the same time point j). The greater the discrepancy (i.e. difference), the lower the weighting/contribution of the initial similarity measure to the measure of similarity, (i.e., the lower $Y_j$).

Thus, in the first example approach for performing the similarity measure generation process 301, the simulated image data contains at least one simulated representation of the one or more interventional devices. Accordingly, the received image data for comparing to said simulated image data comprises at least one representation of the interventional device within the portion of the anatomical cavity.

A second example approach for performing the similarity measure generation process 301 is hereafter described.

In this second example approach, the plurality of points are processed to produce an anatomical model of a portion of the anatomical cavity. This can be performed using any previously described approach, e.g., processing the plurality of points to generate a mesh representing the anatomical cavity.

In this second example approach, the image data received in step 310 comprises a representation of the portion of the anatomical cavity. Thus, for the second example approach, the image data does not need to comprise a representation of the interventional device within the anatomical cavity.

Step 330 may comprise generating the simulated image data based on the model of the anatomical cavity. This can be performed using any suitable model-to-image simulation technique. In particular, the anatomical model can be processed to produce simulated image data of the portion of the anatomical cavity represented in the image data.

Examples of suitable techniques for simulating image data from a model are known in the art, and can include ray casting algorithms. Example approaches for generating image data from an anatomical model are disclosed by the following example documents: Segars, William Paul, et al. "Realistic CT simulation using the 4D XCAT phantom." Medical physics 35.8 (2008): 3800-3808; Lane, Jeffrey M., and Richard F. Riesenfeld. "A theoretical development for the computer generation and display of piecewise polynomial surfaces." IEEE Transactions on Pattern Analysis and Machine Intelligence 1 (1980): 35-4; and Freud, Nicolas, et al. "Fast and robust ray casting algorithms for virtual X-ray imaging." Nuclear Instruments and Methods in Physics Research Section B: Beam Interactions with Materials and Atoms 248.1 (2006): 175-180.

For the second example approach, step 340 can be carried out using any previously described approach (i.e., using any image similarity function).

In a modified version of the second example approach, a plurality of instances of image data are received in step 310. A corresponding plurality of instances of simulated image data are generated in step 330 (each corresponding to a respective instance of image data and representing a same imaged region). Step 340 may comprise determining a cumulative measure of similarity between each instance of image data and its corresponding simulated image data.

The second example approach provides a technique by which a comparison is made between the simulated representation of the portion of the anatomical cavity and the true representation of the same, contained in the image data.

Generic approaches for performing the mapping function adjustment process 302, i.e., step 350, using an error value provided by a cost/loss function are established in the art. Generally, such approaches comprise modifying a mapping function, particularly one or more parameters of the mapping function, to reduce or minimize this error value.

For instance, a gradient descent approach can be used to iteratively modify the mapping function, with the effect of the iterative modification being reassessed each time (by monitoring the impact on the error value provided by the cost function).

In some examples, the measure of similarity alone is sufficient to act as an error value for the purposes of step 302, 350. Thus, step 302, 350 may comprise modifying the mapping function to reduce or minimize the value of the measure of similarity alone.

In some examples, multiple (different) measures of similarity are generated are combined to act as the error value for the purposes of step 302, 350. Different measures of similarity can be produced, for example, by using different image similarity functions to generate different measures of similarity.

However, in preferred examples, the error value which is minimized or reduced during the mapping function adjustment process 302, 350 is defined using a combination of the measure of similarity (or a plurality of measures of similarity) and at least one other cost function output. A cost function output is a value that represents some other indication of an error or inaccuracy of the mapping function.

Thus, the step 350 of adjusting the mapping function may comprise adjusting the mapping function to minimize or reduce an error value calculated or determined by combining the measure(s) of similarity and the at least one other cost function output.

As an example, the at least one other cost function output may comprise or be a cost function output that is responsive to, for each of one or more pairs of points in the spatial co-ordinate system, a difference between the distance in the spatial co-ordinate system between the pair of points (i.e., an estimated inter-sensor distance) and a known distance between the pair of points (i.e., a known inter-sensor distance).

In effect, this cost function output can represent differences between known inter-sensor distances and the estimated inter-sensor distances in the spatial co-ordinate system. This is an indication of the error or accuracy or the mapping function.

Put another way, each pair of points may be a pair of points derived using the mapping function from measurements, of the crossing electromagnetic fields, sampled by a pair of field-sensitive sensors at a same time, wherein and a distance between the pair of field-sensitive sensors is predetermined (i.e., is a known distance).

The cost function output may therefore be the conventional cost function output used for adjusting or modifying the mapping function

Mathematically, this example of a cost function output $A(\theta)$ is represented by the following equation:

$$A(\theta) = \sum_{i \in s} \sum_{k,l \in p} \left( \left| f(\mathbf{x}_k^i; \theta) - f(\mathbf{x}_l^i; \theta) \right| - \Delta_{k,l} \right)^2 \qquad (2)$$

wherein:

s is the index set of all positions of the interventional device at which measurements are captured by the field-sensitive sensors carried by said interventional device;

p is the index set of all pairs of the field-sensitive sensors;

k is one field-sensitive sensor in a pair;

$\mathbf{x}_k^i$ is the measurement captured by the k-th field sensitive sensor at position i of the interventional device;

1 is the other field-sensitive in the pair;

$\mathbf{x}_l^i$ is the measurement captured by the l-th field-sensitive sensor at position i of the interventional device;

f(.) is the mapping function;

θ is the parameterization of the mapping function; and

$\Delta_{k,l}$ is the known distance between the k-th field sensitive sensor and the 1-th field-sensitive sensor.

The step of adjusting the mapping function may comprise adjusting the mapping function responsive to an error value defined by a combination of the measure(s) of similarity and the cost function output. The combination may, for instance, be a summation or a multiplication of the measure of similarity and the cost function output.

In some examples, the combination of the measure of similarity and the cost function output (used in producing the error value) is a weighted combination. Thus, step 302 may comprise combining the measure of similarity and the cost function output using a weighted combination, such as a weighted sum or weighted average.

As an example, the error value may be represented by the following equation:

$$A(\theta) + \lambda\, B(\theta) + C \qquad\qquad (3)$$

where λ is a weighting parameter. The value of the weighting parameter (i.e., the weighting) may be predetermined or known in advance.

C represents one or more regularization terms or parameters. These are optional (i.e., C may equal 0). The regularization term(s) or parameter(s) can be used to make the mapping function adjustment more robust or biased towards certain solutions, such as those known to be suitable. Such regularization parameter(s) may be combined with the measure(s) of similarity to form an error function, even in embodiments that do not make use of the cost function output A(θ).

Of course, equation (3) assumes that there is a single measure of similarity. If there are a plurality of measures of similarity, then the value B(θ) may be replaced by a combination of the measures of similarity, e.g., summation or averaging of the measures of similarity.

The combination of the measures of similarity, if used, may be a weighted combination (e.g., to weight certain measures more heavily than other measures).

The value of the weighting parameter (i.e., the weighting) provided to the measure(s) of similarity may be configured to increase responsive to the number of the pairs of points in the spatial co-ordinate system being below a predetermined threshold number. Thus, the influence of the measure(s) of similarity on the error value may increase responsive to the number of the pairs of points in the spatial co-ordinate system being below a predetermined threshold number.

By way of example, if used, the value of the weighting parameter λ in equation (3) may increase responsive to the number of the pairs of points in the spatial co-ordinate system being below a predetermined threshold number. The predetermined threshold number may, for instance, be a value no greater than 50, e.g., no greater than 25.

As another example, the value of the weighting parameter (i.e., the weighting) provided to the measure of similarity may be configured to reduce responsive to a time difference increasing further comprising receiving a time difference. A time difference is a difference between a time at which the plurality of points (for a particular measure of similarity) was originally sampled and a time at which the image data was originally obtained.

As yet another example, the value of the weighting parameter (i.e., the weighting) provided to the measure of similarity may be configured to be responsive to a user input. This provides a user with a mechanism for controlling the level of reliance upon the comparison with image data.

As yet another example, the value of the weighting parameter (i.e., the weighting) may be configured to reduce responsive to the number of different recorded orientations and/or the distribution of recorded orientations of the interventional device (carrying the one or more field-sensitive sensors) increasing. Each recorded orientation may represent a different angle that the interventional device makes with respect to the anatomical cavity. The larger the number of orientations, the greater the accuracy of modifying the mapping function using known distances between field-sensitive sensors, and the less reliance upon the image data comparison technique is required.

As previously explained, if multiple measures of similarity are used, then there may be a weighted combination of the multiple measures of similarity. In this approach, the weighting to each measure of similarity may be responsive to a respective time difference, being a difference between a time at which the plurality of points (used in generating that measure of similarity) was originally sampled and a time at which the image data (used in generating that measure of similarity) was originally obtained. In particular, the weighting provided to the measure of similarity is configured to reduce responsive to the time difference increasing.

As previously mentioned, a time difference can be determined using appropriate timestamps.

It will be appreciated that, if an anatomical model is generated from the plurality of points, the proposed approach generates an intrinsic registration of an anatomical model and the image data, as a spatial relationship between the anatomical model and the image data is defined by the measure of similarity.

Thus, in some examples, the method may further comprise spatially registering the anatomical model and the image data responsive to the measure of similarity.

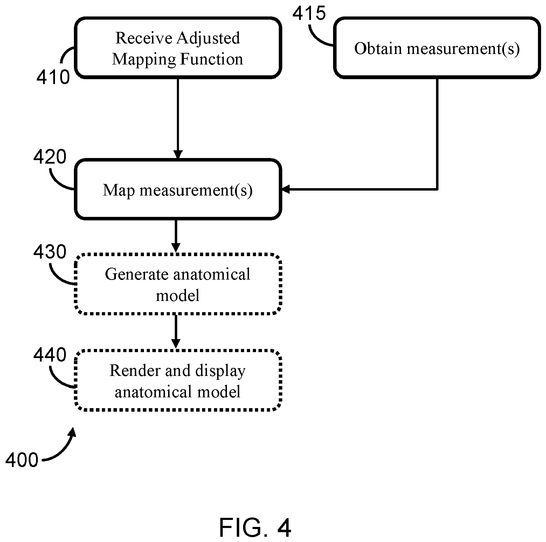

Fig. 4 illustrates a method 400 according to an embodiment. The method is for mapping a measurement, of crossing electromagnetic fields, by a field-sensitive sensor within an anatomical cavity of a subject to a point in a spatial co-ordinate system. With reference to Fig. 1, the method 400 may be performed by at least the mapping system 100.

The method 400 comprises a step 410 of obtaining the mapping function adjusted by performing any previously described method 300. Step 310 may, for instance, comprise receiving the mapping function from a memory or storage unit (or another processing unit) or performing the adjustment to the mapping function, i.e., performing method 300.

The method 400 also comprises a step 420 of mapping the measurement by the field-sensitive sensor to the point in the spatial co-ordinate system using the obtained mapping function. Thus, the measurement may be input to the (adjusted) mapping function which then outputs a corresponding point in the spatial co-ordinate system.

Steps 410, 420 can be performed for a plurality of measurements, e.g., obtained by one or more field-sensitive sensors at one or more different locations/positions of the interventional device upon which they are mounted.

Of course, if required, method 400 may comprise obtaining 415 the measurement(s) to be mapped. This may comprise obtaining the measurements from a memory/database or another processing module.

The method 400 may further comprise generating 430 an anatomical model using the determined/mapped points in the spatial co-ordinate system. Approaches for generating an anatomical model from points in the spatial co-ordinate system have been previously described.

The method 400 may then comprise rendering and displaying the anatomical model, e.g., at a user interface 190. This provides useful information for a clinician or individual about the anatomical cavity.

The skilled person would be readily capable of developing a processing system for carrying out any herein described method. Thus, each step of a flow chart may represent a different action performed by a processing system, and may be performed by a respective module of the processing system.

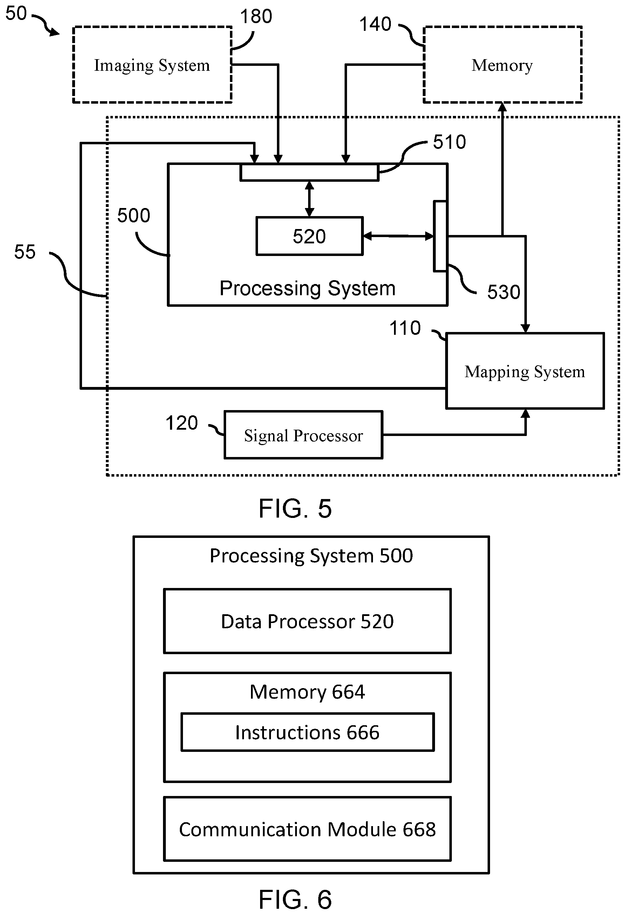

Fig. 5 illustrates one example of a processing system 500 for adjusting a mapping function configured to map from a measurement, of crossing electromagnetic fields, by a field-sensitive sensor within an anatomical cavity of a subject to a point in a spatial co-ordinate system.

Although illustrated as a separate element for the sake of illustrative clarity, in practice, the processing system 500 may be formed as an aspect of the mapping system 110. Thus, there may be provided a mapping system comprising the processing system 500.

The processing system 500 may form part of a larger (computing) system 55, which also provides an embodiment. The computing system 55 may, for instance, be embodied as a workstation or the like. The processing system may also/otherwise form part of a larger, overall system 50, which further includes the imaging system and/or the memory 140.

The processing system 500 comprises an input interface 510 configured to receive image data of a portion of the interventional device and/or a portion of the anatomical cavity. The image data may be received from an imaging system 180 and/or a memory 140.

The input interface 510 is also configured to receive a plurality of points in the spatial co-ordinate system, each point representing a location of a field-sensitive sensor in the spatial co-ordinate system, wherein each point is produced using the mapping function. The plurality of points may be generated by the mapping system, e.g., another component or aspect of the mapping system. Alternatively, the plurality of points may be obtained from a memory 140.

The processing system 500 also comprises a data processor 520 communicatively coupled to the input interface.

The data processor 520 is configured to generate simulated image data of the portion of interventional device and/or the portion of the anatomical cavity by processing the plurality of points in the spatial co-ordinate system; determine a measure of similarity between the image data and the simulated image data; and adjust the mapping function responsive to the determined measure of similarity.

The processing system 500 may be appropriately adapted to perform any herein described method, as would be readily apparent to the skilled person.

The processing system 500 may further comprise an output interface 530, which provides the adjusted mapping function. This information may be output, for instance, to a further data processor 590 such as a further processing module (e.g., of the mapping system). In some examples, the information may be provided to a/the memory 140 or other storage unit.

Fig. 6 is a schematic diagram of a processing system 500, according to embodiments of the present disclosure. The processing system 500 is configured to carry out a method according to an embodiment, such as the method 300 described with reference to Fig. 3.

As shown, the processing system 500 may include a data processor 520, a memory 664, and a communication module 668. These elements may be in direct or indirect communication with each other, for example via one or more buses.

The data processor 520 may include a central data processor (CPU), a digital signal processor (DSP), an ASIC, a controller, an FPGA, another hardware device, a firmware device, or any combination thereof configured to perform the operations described herein. The data processor 520 may also be implemented as a combination of computing devices, e.g., a combination of a DSP and a microprocessor, a plurality of microprocessors, one or more microprocessors in conjunction with a DSP core, or any other such configuration. In some embodiments, the processor is a processing system, e.g., formed of a set of distributed processors.

The memory 664 may include a cache memory (e.g., a cache memory of the data processor 520), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 664 includes a non-transitory computer-readable medium. The non-transitory computer-readable medium may store instructions. For example, the memory 664, or non-transitory computer-readable medium may have program code recorded thereon, the program code including instructions for causing the processing system 500, or one or more components of the processing system 500, particularly the data processor 520, to perform the operations described herein. For example, the processing system 500 can execute operations of the method 300.

Instructions 666 may also be referred to as code or program code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. The memory 664, with the code recorded thereon, may be referred to as a computer program product.

The communication module 668 can include any electronic circuitry and/or logic circuitry to facilitate direct or indirect communication of data between the processing system 500 and any other system. In that regard, the communication module 668 can be an input/output (I/O) device. In some instances, the communication module 668 facilitates direct or indirect communication between various elements of the processing system 500.

The communications module 668 may comprise the input interface 510 and the output interface 530, for facilitating communication between the system 500 and external devices.

It will be understood that disclosed methods are preferably computer-implemented methods. As such, there is also proposed the concept of a computer program comprising computer program code for implementing any described method when said program is run on a processing system, such as a computer or a set of distributed processors.

Different portions, lines or blocks of code of a computer program according to an embodiment may be executed by a processing system or computer to perform any herein described method. In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

The present disclosure proposes a computer program (product) comprising instructions which, when the program is executed by a computer or anatomical model registration system, cause the computer or anatomical model registration system to carry out (the steps of) any herein described method. The computer program (product) may be stored on a non-transitory computer readable medium.

Similarly, there is also proposed a computer-readable (storage) medium comprising instructions which, when executed by a computer or anatomical model registration system, cause the computer or anatomical model registration system to carry out (the steps of) any herein described method. There is also proposed computer-readable data carrier having stored there on the computer program (product) previously described. There is also proposed a data carrier signal carrying the computer program (product) previously described.

It will be appreciated that different disclosed processing systems may form part of a single processing system, e.g., each processing system may be a sub-system of a single processing system. In the context of the present disclosure, this means that a single system could perform the actions of a mapping system and/or a processing system and/or a field controller processing system as herein described.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

A single processor or other unit may fulfill the functions of several items recited in the claims. A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". Any reference signs in the claims should not be construed as limiting the scope.

**Claims**

1. A computer-implemented method of adjusting a mapping function configured to map from a measurement, of crossing electromagnetic fields, by a field-sensitive sensor carried by an interventional device within an anatomical cavity of a subject to a point in a spatial co-ordinate system, the computer-implemented method comprising:

   receiving image data of a portion of the interventional device and/or a portion of the anatomical cavity;
   receiving a plurality of points in the spatial co-ordinate system, each point representing a location of a field-sensitive sensor in the spatial co-ordinate system, wherein each point is produced using the mapping function;
   generating simulated image data of the portion of interventional device and/or the portion of the anatomical cavity by processing the plurality of points in the spatial co-ordinate system;
   determining a measure of similarity between the image data and the simulated image data; and
   adjusting the mapping function responsive to the determined measure of similarity.

2. The computer-implemented method of claim 1, wherein:

   the image data is of the portion of the interventional device; and
   the step of generating simulated image comprises generating simulated image data of the portion of the interventional device.

3. The computer-implemented method of claim 1 or 2, wherein the step of determining a measure of similarity comprises processing the image data and the simulated image data using a cross-correlation technique.

4. The computer-implemented method of claim 3, wherein the cross-correlation technique is a normalized cross-correlation.

5. The computer-implemented method of any of claims 1 to 4, further comprising receiving a cost function output, the cost function output being responsive to, for each of one or more pairs of points in the spatial co-ordinate system, a difference between the distance in the spatial co-ordinate system between the pair of points and a known distance between the pair of points, wherein the step of adjusting the mapping function is further responsive to the cost function output.

6. The computer-implemented method of claim 5, wherein:

   each pair of points is a pair of points derived using the mapping function to process measurements, of the crossing electromagnetic fields, sampled by a pair of field-sensitive sensors at a same time; and
   a distance between the pair of field-sensitive sensors is predetermined.

7. The computer-implemented method of claim 5 or 6, wherein the step of adjusting the mapping function comprises adjusting the mapping function responsive to a combination of the measure of similarity and the cost function output.

8. The computer-implemented method of claim 7, wherein the step of adjusting the mapping function comprises adjusting the mapping function to minimize or reduce the value of the combination of the measure of similarity and the cost function output.

9. The computer-implemented method of any of claims 7 or 8, wherein the combination of the measure of similarity and the cost function output is a weighted combination.

10. The computer-implemented method of claim 9, wherein the weighting provided to the measure of similarity is configured to increase responsive to the number of the pairs of points in the spatial co-ordinate system being below a predetermined threshold number.

11. The computer-implemented method of any of claim 9 or 10, further comprising receiving a time difference, being a difference between a time at which the plurality of points was originally sampled and a time at which the image data was originally obtained, wherein:
the weighting provided to the measure of similarity is configured to reduce responsive to the time difference increasing.

12. The computer-implemented method of any of claims 1 to 11, further comprising:

receiving a plurality of measurements, of the crossing electromagnetic fields, by one or more field-sensitive sensors within the anatomical cavity;
processing each measurement using the mapping function to produce a plurality of points in the spatial co-ordinate system; and
generating an anatomical model of at least the portion of the anatomical cavity by processing the plurality of points in the spatial co-ordinate system.

13. A computer-implemented method of mapping a measurement, of crossing electromagnetic fields, by a field-sensitive sensor within an anatomical cavity of a subject to a point in a spatial co-ordinate system, the computer-implemented method comprising:

obtaining the mapping function adjusted by performing the method of any of claims 1 to 12; and
mapping the measurement by the field-sensitive sensor to the point in the spatial co-ordinate system using the obtained mapping function.

14. A computer program product comprising code which, when executed by a processor circuit, causes the processor circuit to perform the steps of the method according to claim 13.

15. A processing system for adjusting a mapping function configured to map from a measurement, of crossing electromagnetic fields, by a field-sensitive sensor carried by an interventional device within an anatomical cavity of a subject to a point in a spatial co-ordinate system, the processing system comprising:

an input interface configured to:

receive image data of a portion of the interventional device and/or a portion of the anatomical cavity; and
receive a plurality of points in the spatial co-ordinate system, each point representing a location of a field-sensitive sensor in the spatial co-ordinate system, wherein each point is produced using the mapping function;

a data processor communicatively coupled to the input interface, the data processor being configured to:

generate simulated image data of the portion of interventional device and/or the portion of the anatomical cavity by processing the plurality of points in the spatial co-ordinate system;
determine a measure of similarity between the image data and the simulated image data; and
adjust the mapping function responsive to the determined measure of similarity.

FIG. 1

210

250

220

FIG. 2

301

Receive Image Data 310

Receive Points

320

330 Generate Simulated
Image Data

340

Determine Similarity

Adjust Mapping
Function

350

302

300

FIG. 3

Receive Adjusted
Mapping Function

415

Obtain measurement(s)

410

420

Map measurement(s)

430

Generate anatomical
model

440

Render and display
anatomical model

400

FIG. 4

50

180     140

**Imaging System**

**Memory**

510

500

520

**Processing System**

530

55

110

**Mapping System**

120

Signal Processor

## FIG. 5

Processing System 500

Data Processor 520

Memory 664

Instructions 666

Communication Module 668

## FIG. 6

Europäisches
Patentamt

European
Patent Office

Office européen
des brevets

# EUROPEAN SEARCH REPORT

**Application Number**

EP 22 20 2949

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/034944 A1 (NAVIX INT LTD) 21 February 2019 (2019-02-21) <br> * page 1, paragraph 29-31; figures 1-14 * <br> * page 3, line 10 - line 21 * <br> * page 10, line 23 - page 11, line 2 * <br> * page 11, line 3 - line 15 * <br> * page 24, line 18 - line 32 * <br> * page 38, line 27 - line 32 * <br> * page 48, line 3 - line 20 * <br> * page 57, line 33 - page 58, line 7 * <br> ----- | 14,15 | INV. <br> A61B34/20 |
| X | EP 4 018 925 A1 (KONINKLIJKE PHILIPS NV [NL]) 29 June 2022 (2022-06-29) <br> * the whole document * <br> ----- | 14,15 | |

**TECHNICAL FIELDS SEARCHED (IPC)**

A61B

The present search report has been drawn up for all claims

| Place of search | Date of completion of the search | Examiner |
|---|---|---|
| Munich | 3 March 2023 | Nemchand, Jaya |

## ANNEX TO THE EUROPEAN SEARCH REPORT
## ON EUROPEAN PATENT APPLICATION NO.

EP 22 20 2949

This annex lists the patent family members relating to the patent documents cited in the above-mentioned European search report.
The members are as contained in the European Patent Office EDP file on
The European Patent Office is in no way liable for these particulars which are merely given for the purpose of information.

03-03-2023

| Patent document cited in search report | | Publication date | Patent family member(s) | | Publication date |
|---|---|---|---|---|---|
| WO 2019034944 | A1 | 21-02-2019 | CN 111163692 A | | 15-05-2020 |
| | | | EP 3668393 A1 | | 24-06-2020 |
| | | | WO 2019034944 A1 | | 21-02-2019 |
| EP 4018925 | A1 | 29-06-2022 | EP 4018925 A1 | | 29-06-2022 |
| | | | WO 2022136075 A1 | | 30-06-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3568068 A1
- EP 0775466 A2
- EP 3607879 A1

**Non-patent literature cited in the description**

- **BERGER, MATTHEW et al.** A survey of surface reconstruction from point clouds. *Computer Graphics Forum,* 2017, vol. 26 (1
- Measures of similarity. **UNNIKRISHNAN, RANJITH ; MARTIAL HEBERT.** Seventh IEEE Workshops on Applications of Computer Vision (WACV/MOTION'05)-Volume 1. IEEE, 2005, vol. 1
- Measuring image similarity in the presence of noise. **ROHDE, GUSTAVO KUNDE ; CARLOS A. BERENSTEIN ; DENNIS M. HEALY JR.** Medical Imaging 2005: Image Processing. SPIE, 2005, vol. 5747
- Similarity measures between images. **VAN HEEL, MARIN.** Ultramicroscopy. 1987, vol. 21, 95-100
- **HAASE, CHRISTIAN et al.** Model based 3D CS-catheter tracking from 2D X-ray projections: Binary versus attenuation models. *Computerized medical imaging and graphics,* 2014, vol. 38 (3), 224-231
- **SEGARS, WILLIAM PAUL et al.** Realistic CT simulation using the 4D XCAT phantom. *Medical physics,* 2008, vol. 35 (8), 3800-3808
- **LANE, JEFFREY M. ; RICHARD F. RIESENFELD.** A theoretical development for the computer generation and display of piecewise polynomial surfaces. *IEEE Transactions on Pattern Analysis and Machine Intelligence,* 1980, vol. 1, 35-4
- **FREUD, NICOLAS et al.** Fast and robust ray casting algorithms for virtual X-ray imaging. *Nuclear Instruments and Methods in Physics Research Section B: Beam Interactions with Materials and Atoms,* 2006, vol. 248 (1), 175-180